# EUROPEAN PATENT APPLICATION

(11) **EP 4 850 746 A2**
(43) Date of publication of application: **09.12.2026**
(21) Application number: 26209258.8
(22) Date of filing: 22.03.2021
(51) Int. Cl.: A61M 60/825

(54) **BLOOD PUMP**

(30) Priority: 27.03.2020 EP 20166348
(62) Document number(s) of the earlier application(s) in accordance with Art. 76 EPC: 24206413.7 / 4 470 598; 21712841.2 / 3 914 334
(71) Applicant: Abiomed Europe GmbH, 52074 Aachen (DE)
(72) Inventor: KERKHOFFS, Wolfgang, Aachen (DE); KEYSSELITZ, Ellen, Aachen (DE)
(74) Representative: Klunker IP Patentanwälte PartG mbB

(57) **Abstract**

This invention relates to an intravascular blood pump, comprising a pumping device (11) with a pump casing (2) having a primary blood flow inlet (211) and a primary blood flow outlet (22) which are hydraulically connected by a primary blood flow passage (30), a primary impeller (31) with an upstream end and a downstream end being configured to convey a primary blood flow (1BF) from the primary blood flow inlet (211) to the primary blood flow outlet (22) along the primary blood flow passage (30), a drive unit (4) configured to rotate the primary impeller (31) about an axis of rotation (10), an impeller bearing (37) supporting the upstream end of the primary impeller (31), a central opening (262) extending axially through the impeller bearing (37), and at least one secondary blood flow passage (321) in the primary impeller (31), the at least one secondary blood flow passage (321) having a secondary blood flow inlet (212) in axial alignment with the central opening (262) of the impeller bearing (37) and each of the at least one secondary blood flow passage (321) having a secondary blood flow outlet (213), wherein at least one secondary blood flow passage (321) is configured to convey a secondary blood flow (2BF) from the secondary blood flow inlet (212) to the secondary blood flow outlet (213), and wherein the secondary blood flow outlet (213) connects the at least one secondary blood flow passage (321) to the primary blood flow passage (30) at a location axially between the upstream and downstream ends of the primary impeller (31).

## Description

### TECHNICAL FIELD OF THE INVENTION

This invention relates to an intravascular blood pump to support or replace the function of the heart by creating an extra blood flow in a patient's blood vessel.

### BACKGROUND OF THE INVENTION

Blood pumps of different types are known such, as axial blood pumps, centrifugal blood pumps and mixed-type or diagonal blood pumps, where the blood flow is caused by both axial and radial forces. Intravascular blood pumps are usually inserted percutaneously, such as through the femoral artery into the left ventricle so as to bridge the aortic valve or through the femoral vein into the right ventricle.

A rotary blood pump has an axis of rotation. In this patent application, the terms "radial" and "axial" refer to the axis of rotation and mean "in radial direction in relation to the axis of rotation" and "along the axis of rotation", respectively. The term "inner" means radially toward the axis of rotation, and the term "outer" means radially away from the axis of rotation.

An intravascular blood pump typically comprises a pumping device as a main component. The pumping device has a pump section including a primary impeller for pumping the blood from a blood flow inlet to a blood flow outlet and a drive section including a motor for driving the primary impeller. The pump section may include a flexibly bendable cannula between the blood flow inlet and outlet.

The pumping device comprises a pump section end which is arranged at a pump side of the pumping device. The pumping device further comprises a drive section end which is arranged at a drive side of the pumping device. The blood pump may further comprise a catheter connected to the pumping device in order to supply the pumping device e.g. with energy, and/or a purge fluid. The catheter may be connected to the pump section end but is mostly connected to the drive section end of the pumping device. It is also conceivable to rotate the impeller in a forward and in a reverse direction. Then, the blood flow inlet and the blood flow outlet of the pump section may interchange.

Usually, the impeller is supported within the pumping device by means of at least one impeller bearing. Different rotor bearing types are known, such as sliding bearings, in particular hydrodynamic sliding bearings, pivot bearings, hydrostatic bearings, ball bearings etc., and combinations thereof. In particular, contact-type bearings may be realized as "blood-immersed bearings", where the bearing surfaces have blood contact. Problems during operation may be friction and heat. In case of a blood-immersed bearing, a further problem may be blood clotting due to heat or not enough rinse.

An example for blood-purged radial sliding rotor bearings is disclosed in WO 2017/021465 which describes an intravascular blood pump comprising a generally cylindrical primary impeller and a generally cylindrical secondary impeller which rotate together. The secondary impeller is arranged at the radial center of the primary impeller. Vanes of the secondary impeller extend toward an axis of rotation of both impellers. The tips of the vanes of the secondary impeller form an outer bearing surface of a sliding bearing. A cylindrical outer surface of a pin which is arranged at the center of the secondary impeller forms the inner bearing surface of the sliding bearing. In another embodiment, blood from the center of an arriving blood stream can enter the blood pump through a central axial passage in the impeller. In all embodiments the primary and the secondary impeller are mounted on a non-rotating central pin. This increases the hydraulic resistance of the secondary blood flow.

### SUMMARY OF THE INVENTION

It is an object of the present invention to provide a blood pump with reduced hydraulic resistance for the pumped blood.

This is achieved according to the present invention by a blood pump having the features of independent claim 1. Preferred embodiments and further developments of the invention are specified in the claims dependent thereon.

According to a first aspect of the invention, an intravascular blood pump comprises a pumping device with a pump casing having a primary blood flow inlet and a primary blood flow outlet which are hydraulically connected by a primary blood flow passage, wherein a primary impeller has an upstream end and a downstream end and is configured to convey a primary blood flow from the primary blood flow inlet to the primary blood flow outlet along the primary blood flow passage. The pumping device further comprises a drive unit configured to rotate the primary impeller about an axis of rotation. An impeller bearing supports the upstream end of the primary impeller, wherein a central opening axially extends through the impeller bearing. The pumping device further comprises at least one secondary blood flow passage in the primary impeller, the at least one secondary blood flow passage having a secondary blood flow inlet in axial alignment with the central opening of the impeller bearing. Each secondary blood flow passage has a secondary blood flow outlet and is configured to convey a secondary blood flow from the secondary blood flow inlet to the secondary blood flow outlet. The secondary blood flow outlet connects the at least one secondary blood flow passage to the primary blood flow passage at a location axially between the upstream and downstream ends of the primary impeller.

In other words, the secondary blood flow passage or passages extend diagonally through the primary impeller, starting centrally at the distal tip of the impeller and terminating in a lateral surface of the impeller so that the secondary blood flow is taken from the center of the arriving blood stream, where the blood stream is fastest and has the most kinetic energy, and meets and supports the primary blood flow in the primary blood flow passage.

Due to their diagonal extension, the secondary blood flow passages also generate pressure in the secondary blood flow by centrifugal forces. Thus, blood can be pumped through the intravascular blood pump with greater ease.

Thus, the secondary blood flow outlet is not arranged at the downstream end of the primary impeller. In such a case, the secondary blood flow would mix with the primary blood flow downstream beyond the primary impeller. This would require a comparably long secondary blood flow passage with increased hydraulic resistance.

A further advantage of the construction is that no stationary parts are present along the secondary blood flow passage. This reduces the hydraulic resistance of the blood pump.

The velocity of the blood can be utilized as it is not decelerated by a stationary pin bearing as according to the state of the art.

The one or more secondary blood flow passages can be considered to constitute a secondary impeller within the primary impeller. The primary and secondary impeller rotate together. The secondary impeller may be realized completely or partly as an inlay part in the tip end of the primary impeller.

It is preferred that at least a part of the secondary impeller is arranged inside the central opening. Then, arriving blood can immediately be conveyed by the secondary impeller. The impeller bearing can be arranged at an outer circumference of the secondary impeller.

The central opening may define the outer impeller bearing surface of the impeller bearing. Inside the central opening, a part of the primary or the secondary impeller may be arranged and form a corresponding inner impeller bearing surface. Preferably, at least one secondary blood flow passage extends into the central opening. For example, the inner impeller bearing surface may be formed by the outer circumference of one or more secondary impeller vanes defined by the secondary blood flow passages.

It is, however, also possible that the inner impeller bearing surface is arranged at the outer circumference of a part of the primary impeller which is not the secondary impeller. This part can be arranged inside the central opening.

Preferably, the primary blood flow inlet is separated from the secondary blood flow inlet by an inflow separator. The inflow separator preferably has the form of a ring. Thus, blood that arrives at the blood pump is divided and flows into the primary or into the secondary blood flow inlet. Preferably, the inflow separator is stationary. Then, the inflow separator can form the outer impeller bearing surface of the impeller bearing. Alternatively, an outer surface of the inflow separator may form an inner impeller bearing surface of the impeller bearing. The impeller bearing radially supports the primary impeller. It is possible that the primary impeller is mounted on the impeller bearing by the secondary impeller or a part thereof.

The inflow separator may comprise an additional impeller bearing ring as a separate component. The impeller bearing ring may form the outer or inner impeller bearing surface of the impeller bearing, but it is preferably arranged on the inside of the inflow separator to form an inner impeller bearing surface. The impeller bearing ring may be made of a material that is different from the material of the inflow separator. Particularly, the impeller bearing ring may be made of a ceramic material, especially of silicon carbide.

The inflow separator may be supported by at least one strut that connects the inflow separator with the pump casing. Preferably, a minimum of three struts is provided. The struts may extend across the primary blood flow inlet. It is preferred that the struts are configured with a low hydraulic resistance.

The inflow separator preferably comprises at least one cut-out at a downstream end of the inflow separator. Preferably, the cut-out has a circumferential width that is comparable to a circumferential width of a secondary blood flow passage. This way, the rotational position of the primary impeller may be defined by at least one secondary blood flow passage extending into and matching the circumferential position of the cut-out. Then, blood clots that begin to build up on the inner (or outer) impeller bearing surface can be removed by an edge of the cut-out when the inner (or outer) impeller bearing surface rotates over it. In case that the corresponding outer (or inner) impeller bearing surface is discontinuous, such as the tip ends of the vanes defined by the second blood flow passages of the secondary impeller, the outer (or inner) impeller bearing surface at the inflow separator can be cleaned by an edge of such vanes in a similar way. A further advantage of the cut-outs is that the cleaning edges of the cut-outs can be flushed by blood flowing through the cut-outs such that blood clots or debris do not accumulate. An inner or outer impeller bearing surface formed by the primary or secondary impeller preferably overlaps the cut-outs axially such that the whole impeller bearing surface of the primary or secondary impeller will be cleaned. Preferably, the outer impeller bearing surface of the inflow separator preferably overlaps axially with the end surfaces of the vanes of the secondary impeller, which form the inner impeller bearing surface, such that the whole outer impeller bearing surface will be cleaned. It is preferred that the tips of the vanes of the secondary impeller have a greater axial length than the circumferential length of the cut-outs. Preferably, at least one cut-out extends between two struts.

The cut-out is preferably not only arranged in the inflow separator, but extends through the aforementioned impeller bearing ring which may be present optionally. Then, the cut-out is completely open on both sides such that the cut-out can be washed effectively.

The impeller bearing may be a sliding bearing. Preferably, the impeller bearing is a blood-purged sliding bearing. This has the advantages that the blood from the blood stream can be used to purge the bearing and, thereby, also cool the bearing.

Preferably, a center of area in a mathematical sense, meaning the middle of a certain region, of the primary blood flow inlet is arranged in the secondary blood flow inlet. The primary blood flow inlet is arranged around the secondary blood flow inlet such that the middle of a blood stream that arrives at the blood pump enters the blood pump through the secondary blood flow inlet. The middle of a laminar blood stream has the highest velocity and is, thus, supplied to the secondary blood flow.

After the blood has passed the primary and secondary blood flow inlet, it enters the primary impeller and secondary impeller, respectively, at respective primary and secondary channel intakes into the primary and secondary blood flow passages. Preferably, at least one secondary channel intake is arranged at a position of the axis of rotation. Usually, the tip end of an impeller does not have a rational velocity so that blood may clot by adhesion and accumulation may arise on a stationary solid tip end. However, with the proposed arrangement, the middle part of the bloodstream flows into the intakes of the secondary blood flow passages. Thus, blood clotting cannot occur.

The secondary channel intakes are preferably arranged upstream of the primary channel intakes. An end of the secondary impeller may then be arranged inside the impeller bearing.

The primary impeller comprises at least one blade having a primary pitch at the upstream end of the primary impeller and a secondary blood flow passage having a secondary pitch, the secondary pitch being approximately or exactly the same as the primary pitch. The pitches may deviate from each other as much as necessary to prevent undesirable flow conditions, such as turbulences.

Preferably, at least two of the at least one secondary blood flow passages are arranged asymmetrically in regard to the axis of rotation. This renders it possible to arrange a secondary channel intake at the axis of rotation.

Preferably, two secondary blood flow passages are arranged opposite to each other in regard to the axis of rotation. This enables a compact design of the secondary impeller.

Preferably, as already mentioned, a secondary blood flow passage defines an edge moving over the outer impeller bearing surface upon rotation of the secondary impeller so as to clean the outer impeller bearing surface. The edge acts as a wiper for the outer impeller bearing surface, and blood clots or debris can be removed in this way.

As also briefly mentioned above, the primary impeller may comprise an inlay in which the at least one secondary blood flow passage is formed. Then, the secondary impeller may be made of another material as the primary impeller. For example, the secondary impeller may be made of a ceramic material. This is advantageous for the inner impeller bearing surface. The primary impeller and the secondary impeller may alternatively form one integral piece.

### BRIEF DESCRIPTION OF THE DRAWINGS

The aforegoing summary as well as the following detailed description of preferred embodiments will be better understood when read in conjunction with the appendant drawings. The scope of the disclosure is not limited, however, to the specific embodiments disclosed in the drawings. In the drawings:
Fig. 1 shows a cross section of a first embodiment of a blood pump according to the invention,
Fig. 2 shows a part of Fig. 1 with the pump section in an enlarged view,
Fig. 3 shows a part of Fig. 1 with the drive section in an enlarged view,
Fig. 4 shows a perspective view toward the pump section end of the first embodiment of the blood pump,
Fig. 5 essentially shows the view of Fig. 4, but with a transparent pump casing,
Fig. 6 essentially shows the same view as Fig. 5, but of a second embodiment of the blood pump,
Fig. 7 shows a perspective view of an embodiment of the secondary impeller,
Fig. 8 shows a perspective view of a separator ring of the first embodiment of the blood pump,
Fig. 9 shows a perspective view of a separator ring of the second embodiment of the blood pump,
Fig. 10 shows a cross section of the drive section end of the blood pump in a perspective view depicting an ancillary impeller,
Fig. 11A shows a perspective view of the ancillary impeller and of a rotor bearing ring,

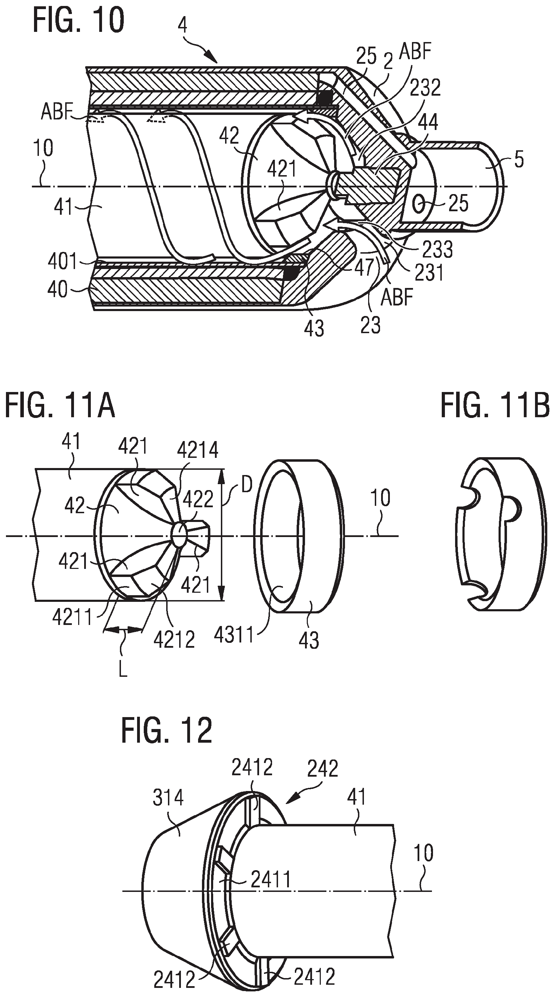

Fig. 11Bshows a perspective view of a rotor bearing ring having cut-outs, and
Fig. 12 shows a perspective view of a tertiary impeller of the first or the second embodiment.

### DETAILED DESCRIPTION

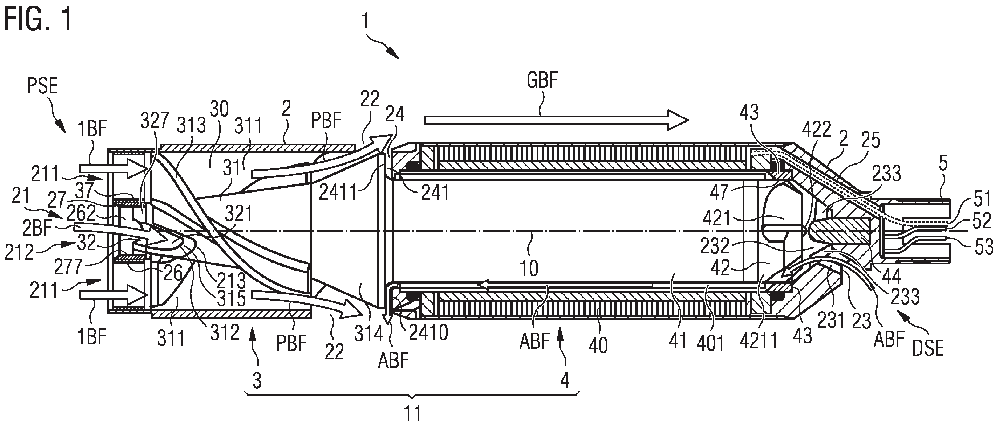

In Fig. 1, a cross sectional view of a first embodiment of an intravascular blood pump is illustrated. Rotating parts are not shown cut. The intravascular blood pump 1 comprises a pumping device 11 and a supply line in the form of a catheter 5 attached thereto.

The pumping device 11 comprises a pump casing 2 of substantially cylindrical form, at least in an intermediate section thereof. The pump casing 2 comprises a blood flow inlet 21 and a blood flow outlet 22. In Fig. 1, the pump casing 2 seems to comprise two separate sections, but these sections are either integral or connected to form a single piece.

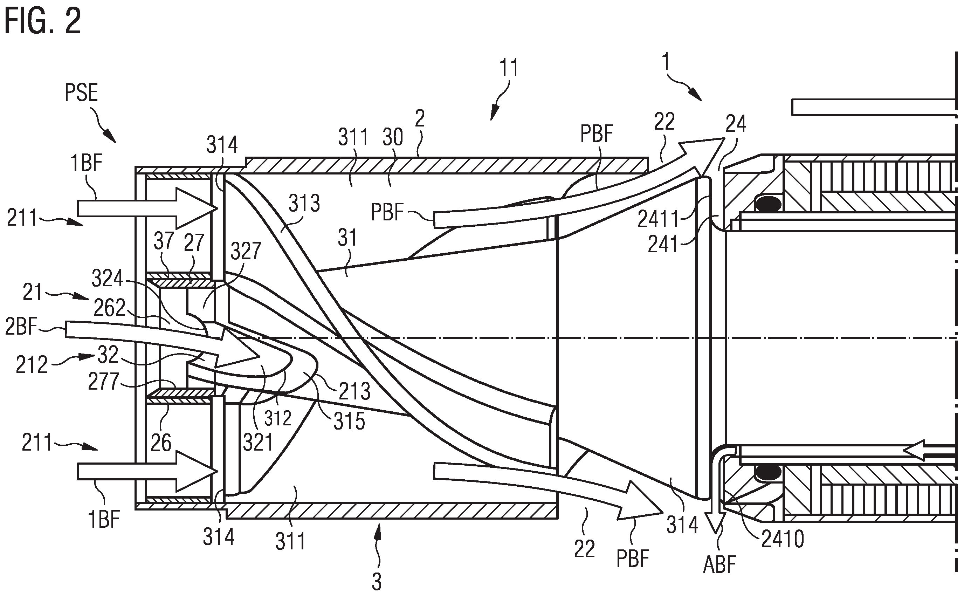

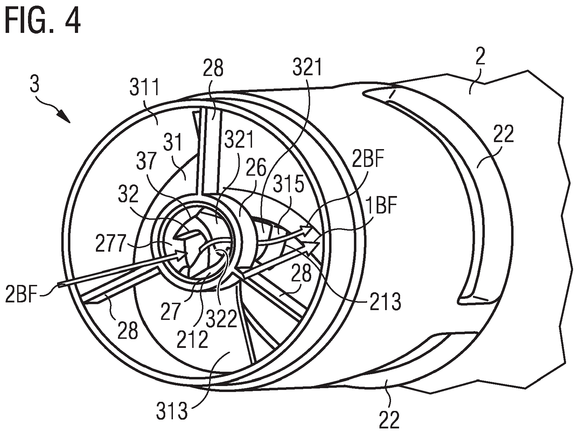

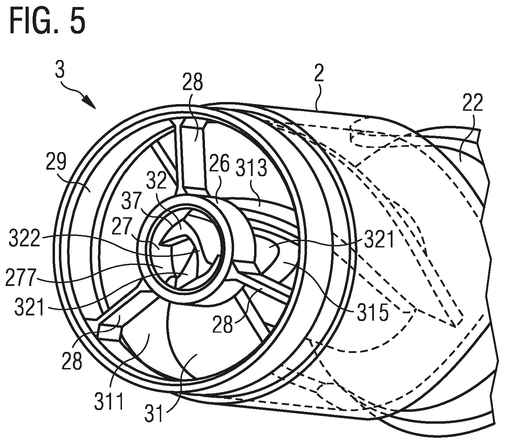

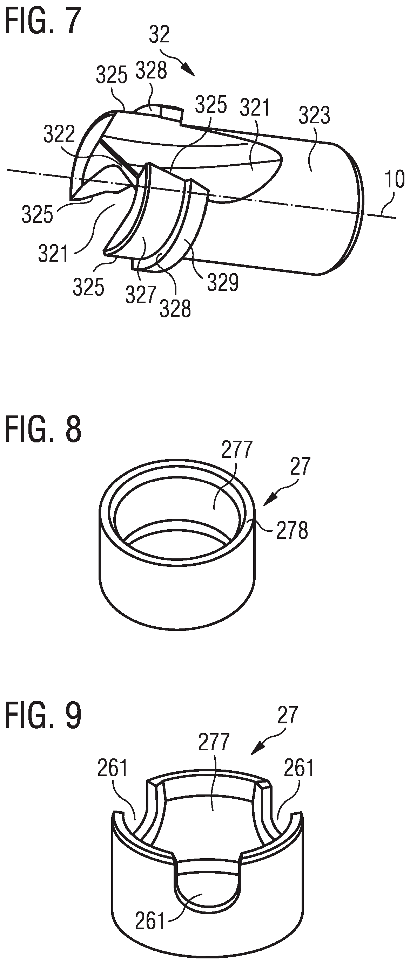

As can be better seen in the enlarged representation of the pump section shown in Fig. 2, together with the front perspective views depicted in Figs. 4 and 5, the blood flow inlet 21 comprises a primary blood flow inlet 211 and a secondary blood flow inlet 212. The primary blood flow inlet 211 surrounds the secondary blood flow inlet 212. The primary blood flow inlet 211 and the secondary blood flow inlet 212 are separated by an inflow separator 26. Inside the inflow separator 26, the inflow separator 26 comprises an impeller bearing ring 27, which is separately shown in Fig. 8. Further, the pumping device 11 comprises a primary impeller 31 which has integrated therein a secondary impeller 32. The primary and secondary impellers 31, 32 are rotatable together about an axis of rotation 10. The secondary impeller 32 may, as shown in Fig. 7, have the form of an inlay and may be arranged inside a secondary impeller cavity 312 of the primary impeller 31. The secondary impeller cavity 312 is open toward a pump section end PSE of the pumping device 11. Alternatively, the primary and secondary impellers 31, 32 are integrally formed.

A primary blood flow 1BF flows from the primary blood flow inlet 211 to the primary impeller 31 outside of the inflow separator 26 to be conveyed further by the primary impeller 31 through a primary blood flow passage 30 to the primary blood flow outlet 22. A secondary blood flow 2BF flows from the secondary blood flow inlet 212 through the inflow separator 26 to the secondary impeller 32 to be conveyed further by the secondary impeller 32 through a plurality of secondary blood flow passages 321 to the primary blood flow passage 30.

Thus, a blood stream arriving at the pumping device 11 at the pump section end, preferably about almost the whole cross section of the pumping device 11, can flow into the primary and secondary blood flow inlets 211, 212 without significant deflection. Because of the central position of the secondary blood flow inlet 212, also blood from the middle of the blood stream can enter the pumping device 11 without deflection. This is advantageous because usually a blood stream is a laminar flow in which the flow velocity is greatest in the center.

The primary impeller 31 comprises primary impeller vanes 313 which extend into the primary blood flow passage 30 and between which primary impeller channels 311 are arranged. The primary impeller channels 311 have a primary pitch at a primary channel intake 314 at each end of the primary impeller channels 311 toward the pump section end PSE. The secondary impeller 32 comprises at least one and particularly exactly two secondary blood flow passages 321 in channel form, which are therefore referred to hereinafter also as secondary impeller channels 321 (see also Fig. 7). The secondary impeller channels 321 have a secondary pitch at a secondary channel intake 324 which is arranged at an upstream end of the secondary impeller channel 321. The secondary pitch is preferably the same as the primary pitch or may vary to a certain degree as long as undesirable flow conditions, such as turbulences, are prevented. At an end of the secondary impeller 32 toward a drive section end DSE, a connecting breakthrough 315 between the secondary impeller cavity 312 and one of the primary blood flow passages 311 is arranged. An end of this breakthrough 315 in the direction of the blood flow defines the secondary blood flow outlet 213. The secondary blood flow outlet 213 is arranged further radially outward in regard to the axis of rotation 10. Therefore, blood is forced outward in a radial direction by centrifugal forces caused by the rotation of the secondary impeller 32. In this way, the secondary blood flow 2BF is conveyed through the secondary blood flow inlet 212 and further through the secondary impeller channel 321 of the secondary impeller 32 and unites with the primary blood flow 1BF flowing through the primary impeller channel 311 of the primary impeller 31. In this way, a pumped blood flow PBF is formed. The pumped blood flow PBF leaves the pumping device 11 at the blood flow outlet 22.

The primary and secondary impellers 31, 32 are jointly mounted in an impeller bearing 37. They are connected via the secondary impeller cavity 312 or integrally formed as one single piece. The inflow separator 26 comprises an impeller bearing ring 27 arranged inside the inflow separator 26. An outer impeller bearing surface 277 of the impeller bearing 37 is arranged at the inside of the impeller bearing ring 27. The impeller bearing 37 further comprises an inner impeller bearing surface 327 which is arranged at an outer circumference of the secondary impeller 32.

The primary impeller 31 is fixedly connected to a tapered section 314 leading to the blood flow outlet 22. The tapered section 314 directs the pumped blood flow PBF in a direction radially outward in regard to the axis of rotation 10. The blood then reaches the blood flow outlet 22.

From the tapered section 314 in a direction toward the pump section end PSE, a drive section 4 is arranged inside the pump casing 2 of the pumping device 11, which comprises a stator 40 and a rotor 41. Between the stator 40 and the rotor 41, an axial gap 401 is arranged. In order to cool the stator 40 and the rotor 41, the axial gap 401 is blood-purged. For this, an ancillary blood flow ABF enters the drive section 4 through an ancillary blood flow inlet 23 arranged at the drive section end DSE. The blood is then conveyed by an ancillary impeller 42 through an ancillary pump gap 423 which is arranged between the ancillary impeller 42 and an inner wall of the pump casing 2. From there, the blood continues to flow into the axial gap 401. From the axial gap 401, the ancillary blood flow ABF enters a radial gap 241. At the radial outer end of the radial gap 241, an ancillary blood flow outlet 24 is arranged. The ancillary blood flow ABF flows in the axially gap 401 in a direction opposite to the pumping direction of the primary and secondary impellers 31, 32. The ancillary blood flow ABF inside the drive section 4 also flows substantially in an opposite direction to a general blood flow GBF flowing around the blood pump 1.

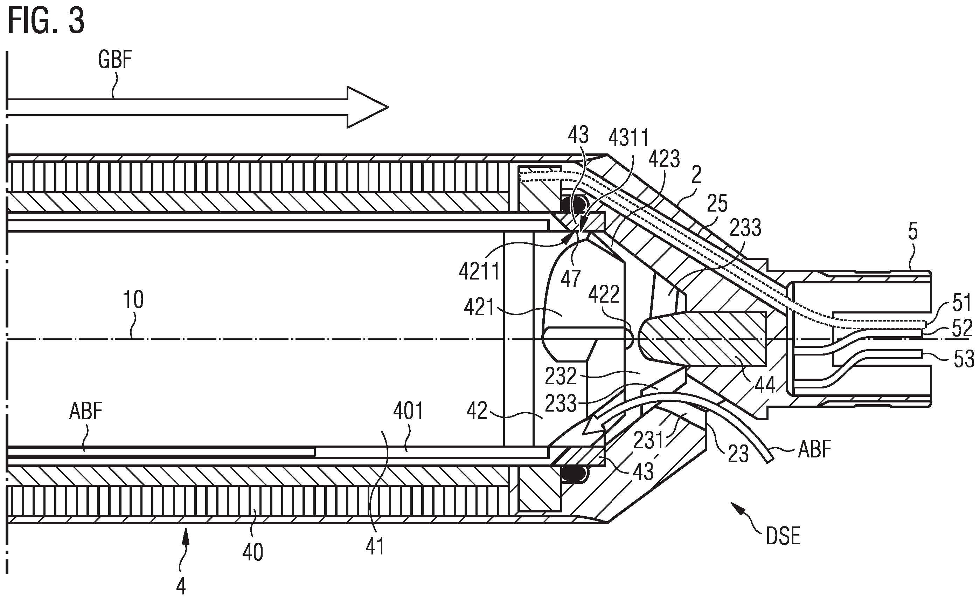

As can be better seen in reference to the enlarged representation shown in Fig. 3, a rotor bearing ring 43 surrounds the ancillary impeller 42. The ancillary impeller 42 comprises ancillary impeller vanes 421. The ancillary impeller vanes 421 protrude in a direction of the axis of rotation 10 toward the drive section end DSE of the pumping device 11. A radial rotor bearing 47 is arranged at the drive section end DSE and comprises an outer rotor bearing surface 4211 and an inner rotor bearing surface 4311, between which an axially extending bearing gap is arranged. The outer rotor bearing surface 4311 is arranged on the rotor bearing ring 43. Blood conveyed by the ancillary impeller 42 flows through the bearing gap and further to the axial gap 401 between the rotor 41 and the stator 40. From the axial gap 401, the blood flows to a radial gap 241. The radial gap 241 extends between the tapered section 314 of the primary impeller 31 and the stator 40. An ancillary blood flow outlet 24 is arranged at the transition between the radial gap 241 and the surrounding of the pumping device 11. The ancillary blood flow outlet 24 is arranged perpendicularly to the axis of rotation 10. Here, the blood from the ancillary blood flow ABF unites with the pumped blood flow PBF from the pump section 2 and the surrounding general blood flow GBF. When the ancillary blood flow outlet 24 is, as shown, arranged close to the outer diameter of the pump casing 2 and close to the primary blood flow outlet 22, the pumped blood flow PBF and the general blood flow GBF support the drawing of blood out of the radial gap 241 because of their flow velocity. This enhances the ancillary blood flow ABF through the axial gap 401.

At a center of the ancillary impeller 42, through which the axis of rotation 10 extends, and at a side of the ancillary impeller 42 opposite to the rotor 41, a hump 422 is arranged. In a direction of the axis of rotation 10 toward the drive section end DSE and adjacent to the hump 422, a bearing pin 44 is arranged. The bearing pin 44 is connected to the pump casing 2. An axial bearing surface of the bearing pin 44 toward the ancillary impeller 42 has a convex shape. The axis of rotation 10 runs through an apex of the axial bearing surface of the bearing pin 44 and through an apex of the axial bearing surface of the hump 422. In this way, the bearing pin 44 interacts with the hump 422 and forms a thrust bearing in order to transmit axial forces in regard to the axis of rotation between the hump 422 and the bearing pin 44, wherein the aforementioned parts are rotatable relative to each other. Obviously, the contact surface is small, such that rotational friction is low.

The drive section end of the blood pump 1 comprises one or more, preferably three, ancillary inlet through-holes 231. The ancillary inlet through-holes 231 extend from the ancillary blood flow inlet 23 to an ancillary impeller cavity 232 in which the ancillary impeller 42 is arranged. Thus, the blood flows from the ancillary blood flow inlet 23 to the ancillary impeller 42 via the ancillary inlet through-hole 231.

At least one wire through-hole 25 is arranged at the drive section end DSE of the pumping device 11. The wire through-holes 25 may extend from the catheter 5 to the stator 40. Preferably, three wire through-holes 25 are arranged about the axis of rotation 10. Between two ancillary inlet through-holes 231, one wire through-hole 25 may be arranged. In a wire through-hole 25, at least one supply line 51, 52 and/or 53 may extend to be connected to the stator 40. Preferably, as shown, the supply wires 51, 52 and/or 53 extend through the inside of the catheter 5 to the outside of the patient's body. The supply wires 51, 52 and/or 53 run from the catheter 5 to the stator 40 without contact to blood.

Fig. 4 shows a perspective front view on the pump section end PSE of the pump section 3. As is shown, the secondary impeller 32 is arranged inside the impeller bearing ring 27. The impeller bearing ring 27 is arranged inside the inflow separator 26. Alternatively to this embodiment, the additional impeller bearing ring 27 can be omitted such that the outer impeller bearing surface 277 is formed by the inflow separator 26. Here, the inflow separator 26 is mounted between the primary blood flow 1BF and the secondary blood flow 2BF by three struts 28. It is shown that the secondary blood flow 2BF flows into the secondary impeller 32 through the secondary blood flow inlet 212 which is arranged at an inflow into the impeller bearing ring 27. In the secondary impeller 32, the blood flows along the secondary impeller channel 321 and through the through-opening 315 to the secondary blood flow outlet 213. Here, the secondary blood flow 2BF unites with the primary blood flow 1BF to form the pumped blood flow PBF.

Fig. 5 shows the pump section end PSE of the pump section 3 in a perspective view, wherein the pump casing 2 is shown in transparency. It is visible that through-opening 315 and the secondary blood flow outlet 213 are arranged between two primary impeller vanes 313. As shown, the struts 28 are connected by an outer strut connection ring 29. The strut connection ring 29 is arranged inside an inner circumferential surface of the pump casing 2 at the pump section end PSE. The impeller bearing ring 27 is supported by the struts 28. It is conceivable to manufacture the strut connection ring 29 and the struts 28 as one piece. Preferably, also the impeller bearing ring 27 is a part of this piece. Said piece may also be formed in one piece with the pump casing 2.

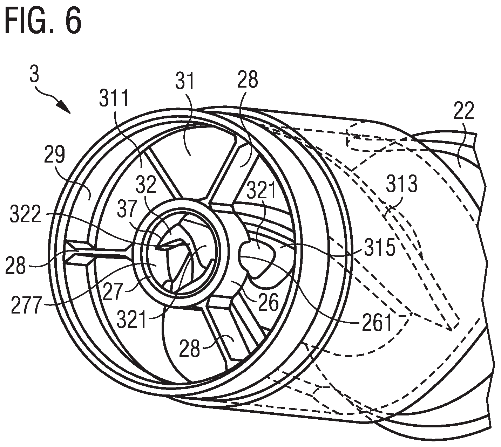

Fig. 6 shows a perspective view of the pump section end PSE of the pump section 3 in which the pump casing 2 is shown in transparency. Different from the embodiment shown in Figs. 3 to 5, the inflow separator 26 comprises at least one, preferably three, cut-outs 261 at a downstream end of the inflow separator 26. The cut-out 261 is arranged between two struts 28. The impeller bearing ring 27 is part of and fixedly connected to the inflow separator 26, and the cut-out 261 also extends through the impeller bearing ring 27. Due to the cut-out 261, the secondary impeller channel 321 has an increased cross section when it aligns with the cut-out 261 during rotation of the secondary impeller. The secondary impeller 32 extends inside the impeller bearing ring 27 in a direction toward the pump section end PSE maximally up to an end of the cut-out 261. This has the effect that, in operation, an edge of the cut-out 261 runs over the inner impeller bearing surface 327 and removes blood clots at the beginning of their formation or preferably prevents their formation, since the mating part of the rotating axial thrust bearing surface 328 is in direct blood contact at the cut-out 261. This helps to avoid stagnant blood within the axial thrust bearing. Also the inner impeller bearing surface 327 has edges 325, as can be seen in Fig. 7, which have the effect to remove blood clots from the outer impeller bearing surface 277 (Figs. 8 and 9).

Fig. 7 shows the secondary impeller 32 in detail in a perspective view. There, the secondary impeller 32 is configured as an inlay and has roughly the form of a cylinder. It may be made of different material as the primary impeller 31, for instance of a ceramic material. The inlay comprises a cylindrical section 323 which is arranged inside the secondary impeller cavity 312 of the primary impeller 21. A circumferential protrusion 329 forms an axial stop for the secondary impeller 32 in the secondary impeller cavity 312. The inner impeller bearing surface 327 is arranged at an outer circumference of the secondary impeller 32. Two secondary impeller channels 321 are arranged at the end of the secondary impeller 32 toward the pump section end PSE. The secondary impeller channels 321 have their largest cross section at the upstream end of the secondary impeller 32. Thus, the channels 321 decrease in cross section away from the blood flow inlet 21. In this way, blood is directed from a mainly axial direction to an axial-radial direction when it flows through the secondary impeller channel 321.

The secondary impeller channels 321 are arranged asymmetrically in regard to the axis of rotation 10 of the secondary impeller 32. At an end of the secondary impeller 32 directed toward the blood flow inlet 21, the axis of rotation 10 extends through one of the secondary impeller channels 321. In this way, the center of rotation, which is located at the axis of rotation 10, does not coincide with a solid part of the secondary impeller 32. This has the advantage that blood clotting at the center of rotation, where no differential velocity to neighboring blood flow is present, can be avoided.

At the transition between the secondary impeller channels 321 and the inner impeller bearing surface 327, edges 325 are arranged. As mentioned above, such edges 325 serve to push away formations of blood clotting on the outer impeller bearing surface 277. The inner impeller bearing surface 327 provides an inner surface of a radial bearing at the pump section end PSE. The secondary impeller 32 further comprises an axial impeller bearing surface 328. It is arranged at the circumferential protrusion 329. The axial impeller bearing surface 328 forms a part of the above-mentioned axial stop or axial thrust bearing. The axial stop may be configured as an axial bearing which is capable of transmitting forces from the secondary impeller 32 to the bearing ring 27 during rotation of the impeller. The axial bearing is necessary to counter the axial force which stems from the purging action of the impeller.

Fig. 8 shows an enlarged view of the impeller bearing ring 27. The outer impeller bearing surface 277 is arranged at the inside of the impeller bearing ring 27. The impeller bearing ring 27 comprises an axial bearing ring surface 278. As shown, the axial bearing ring surface 278 may be arranged at an axial end of the impeller bearing ring 27.

Fig. 9 shows a perspective view of an impeller bearing ring 27 according to a further embodiment which differs from the embodiment shown in Fig. 8 in that it comprises the cut-outs 261, as previously mentioned, which are arranged at a downstream end of the impeller bearing ring 27. The number of cut-outs 261 preferably matches the number of struts 28.

Fig. 10 shows a perspective view of a cross section through the drive section end DSE of the drive section 4. Rotating parts are not shown cut. As shown, the ancillary blood flow ABF enters the pump casing 2 at the ancillary blood flow inlet 23. The ancillary impeller 42 accelerates the blood, which continues to flow into the axial gap 401. As is shown by the arrow ABF inside the axial gap 401, the blood does not flow directly in the direction of the axis of rotation 10, but rather has a strong circumferential flow component so that it flows along the axial gap 401 along helices.

Fig. 11 shows a perspective view of an end of the rotor 41 at the drive section end DSE of the pumping device 11. The ancillary vanes 421 of the ancillary impeller 42 are clearly recognizable, and they extend straight in a radial direction. The ancillary impeller vanes 421 provide, at their outer circumference, the inner rotor bearing surface 4211 of the radial rotor bearing 47. Further, the ancillary impeller vanes 421 each have a chamfer 4212. This chamfer 4212 is advantageous in order to build a tapered drive section end DSE of the pumping device 11 as shown in Fig. 10. Further, the ancillary impeller vanes 421 comprise radially extending end surfaces 4214 at an axial end of the secondary impeller 42. A hump 422 is formed at the center of the axial end of the secondary impeller 42. The hump 422 interacts with the bearing pin 44, as shown in Fig. 10.

Fig. 11A further shows the rotor bearing ring 43 to be arranged around the inner rotor bearing surface 4211 of the secondary impeller 42. The outer rotor bearing surface 4311 of the rotor bearing ring 43 forms the rotor bearing 47 together with the inner rotor bearing surface 4211 of the ancillary impeller 42. The ancillary impeller 42 has an axial Length L and a diameter D. Alternatively, as shown in Fig. 11B, the rotor bearing ring 43 may have cut-outs with a form, function and arrangement similar to the cut-outs 261 of above-described impeller bearing ring 27.

Fig. 12 shows in a perspective view an end of the rotor 41 connected to the tapered section 314 of the primary impeller 31. A tertiary impeller 242 is arranged between the tapered section 314 and the pump section end of the rotor 41, and extends radially from an outer diameter of the rotor 41 to an outer diameter of the tapered section 314 to form a shoulder. An axial plane of this shoulder forms a rotatable wall 2411 of the radial gap 24. From the rotatable wall 2411, the tertiary impeller vanes 2412 project toward the drive section end DSE of the pumping device 11. Preferably, the tertiary impeller vanes 2412 extend axially along the axis of rotation 10. Particularly, the tertiary impeller vanes 2412 are straight and extend in a radial direction relative to the axis of rotation 10. Further in the alternative, the tertiary impeller vanes 2412 can be omitted (not shown).

### Preferred Embodiments of the invention are defined in paras 1 to 20:

1. An intravascular blood pump, comprising a pumping device (11) with
   - a pump casing (2) having a primary blood flow inlet (211) and a primary blood flow outlet (22) which are hydraulically connected by a primary blood flow passage (30),
   - a primary impeller (31) having an upstream end and a downstream end and being configured to convey a primary blood flow (1BF) from the primary blood flow inlet (211) to the primary blood flow outlet (22) along the primary blood flow passage (30),
   - a drive unit (4) configured to rotate the primary impeller (31) about an axis of rotation (10),
   - an impeller bearing (37) supporting the upstream end of the primary impeller (31),
   - a central opening (262) axially extending through the impeller bearing (37),
   - at least one secondary blood flow passage (321) in the primary impeller (31), the at least one secondary blood flow passage (321) having a secondary blood flow inlet (212) in axial alignment with the central opening (262) of the impeller bearing (37) and each of the at least one secondary blood flow passage (321) having a secondary blood flow outlet (213), wherein the at least one secondary blood flow passage (321) is configured to convey a secondary blood flow (2BF) from the secondary blood flow inlet (212) to the secondary blood flow outlet (213), and
   wherein the secondary blood flow outlet (213) connects the at least one secondary blood flow passage (321) to the primary blood flow passage (30) at a location axially between the upstream and downstream ends of the primary impeller (31).
2. The intravascular blood pump according to para 1, wherein the central opening (262) defines an outer impeller bearing surface (277) of the impeller bearing (37), by which the primary impeller (31) is rotatably supported.
3. The intravascular blood pump according to para 1 or 2, wherein the primary blood flow inlet (211) is separated from the secondary blood flow inlet (212) by a non-rotating inflow separator (26), the inflow separator (26) forming the outer impeller bearing surface (277) of the impeller bearing (37).
4. The intravascular blood pump according to para 3, wherein the inflow separator (26) comprises an impeller bearing ring (27) as a separate component forming the outer impeller bearing surface (277) of the impeller bearing (37).
5. The intravascular blood pump according to para 3 or 4, wherein the inflow separator (26) is supported by at least one and preferably three struts (28) extending across the primary blood flow inlet (211).
6. The intravascular blood pump according to para 5, wherein the at least one strut (28) connects the inflow separator (26) to the pump casing (2).
7. The intravascular blood pump according to any one of paras 3 to 6, wherein the inflow separator (26) comprises at least one cut-out (261) at a downstream end of the inflow separator (26).
8. The intravascular blood pump according to any one of paras 3 to 7, wherein the inflow separator (26) has at least one cut-out (261) in the outer impeller bearing surface (277).
9. The intravascular blood pump according to any one of paras 2 to 8, wherein one or more of the at least one secondary blood flow passage (321) defines an edge (325) moving over the outer impeller bearing surface (277) upon rotation of the primary impeller (31) so as to clean the outer impeller bearing surface (277).
10. The intravascular blood pump according to any one of the preceding paras, wherein the impeller bearing (37) is a sliding bearing.
11. The intravascular blood pump according to para 10, wherein the impeller bearing (37) is a blood purged sliding bearing.
12. The intravascular blood pump according to any one of the preceding paras, wherein a center of area of the primary blood flow inlet (211) is arranged in the separated secondary blood flow inlet (212).
13. The intravascular blood pump according to any one of the preceding paras, wherein one secondary blood flow inlet (212) of the at least one secondary blood flow passage (321) is arranged at a position of the axis of rotation (10).
14. The intravascular blood pump according to any one of the preceding paras, wherein a secondary channel intake (324) forming part of the at least one secondary blood flow passage (321) is arranged upstream of a primary channel intake (314) forming part of a primary impeller channel (311) in the primary blood flow passage (30).
15. The intravascular blood pump according to any one of the preceding paras, wherein the primary impeller (31) comprises at least one blade (313) which has a primary pitch at the upstream end of the primary impeller (31) and wherein the at least one secondary blood flow passage (321) has a secondary pitch, the secondary pitch being approximately or exactly the same as the primary pitch.
16. The intravascular blood pump according to any one of the preceding paras, wherein an exactly one of the at least one secondary blood flow passage (321) has an upstream end arranged at a position of the axis of rotation (10).
17. The intravascular blood pump according to any one of the preceding paras, wherein at least two of the at least one secondary blood flow passage (321) are arranged asymmetrically in regard to the axis of rotation (10).
18. The intravascular blood pump according to any one of the preceding paras, wherein two of the at least one secondary blood flow passage (321) are arranged opposite to each other in regard to the axis of rotation (10).
19. The intravascular blood pump according to any one of the preceding paras, comprising exactly two of the at least one secondary blood flow passage (321).
20. The intravascular blood pump according to any one of the preceding paras, wherein the primary impeller (31) comprises an inlay in which the at least one secondary blood flow passage (321) is formed.

## Claims

1. An intravascular blood pump, comprising a pumping device (11) with
- a pump casing (2) having a primary blood flow inlet (211) and a primary blood flow outlet (22) which are hydraulically connected by a primary blood flow passage (30),
- a primary impeller (31) having an upstream end and a downstream end and being configured to convey a primary blood flow (1BF) from the primary blood flow inlet (211) to the primary blood flow outlet (22) along the primary blood flow passage (30),
- an impeller bearing (37) supporting the upstream end of the primary impeller (31),
- a central opening (262) axially extending through the impeller bearing (37),
- at least one secondary blood flow passage (321) in the primary impeller (31), the at least one secondary blood flow passage (321) having a secondary blood flow inlet (212) in axial alignment with the central opening (262) of the impeller bearing (37) and each of the at least one secondary blood flow passage (321) having a secondary blood flow outlet (213), wherein the at least one secondary blood flow passage (321) is configured to convey a secondary blood flow (2BF) from the secondary blood flow inlet (212) to the secondary blood flow outlet (213), and
wherein the secondary blood flow outlet (213) connects the at least one secondary blood flow passage (321) to the primary blood flow passage (30) at a location axially between the upstream and downstream ends of the primary impeller (31).

2. The intravascular blood pump according to claim 1, wherein the central opening (262) defines an outer impeller bearing surface (277) of the impeller bearing (37), by which the primary impeller (31) is rotatably supported.

3. The intravascular blood pump according to claim 1 or 2, wherein the primary blood flow inlet (211) is separated from the secondary blood flow inlet (212) by a non-rotating inflow separator (26), the inflow separator (26) forming the outer impeller bearing surface (277) of the impeller bearing (37), wherein preferably the inflow separator (26) comprises an impeller bearing ring (27) as a separate component forming the outer impeller bearing surface (277) of the impeller bearing (37) and/or the inflow separator (26) is supported by at least one and preferably three struts (28) extending across the primary blood flow inlet (211), wherein more preferably the at least one strut (28) connects the inflow separator (26) to the pump casing (2).

4. The intravascular blood pump according to claim 3, wherein the inflow separator (26) comprises at least one cut-out (261) at a downstream end of the inflow separator (26), and/or, wherein the inflow separator (26) has at least one cut-out (261) in the outer impeller bearing surface (277).

5. The intravascular blood pump according to any one of claims 2 to 4, wherein one or more of the at least one secondary blood flow passage (321) defines an edge (325) moving over the outer impeller bearing surface (277) upon rotation of the primary impeller (31) so as to clean the outer impeller bearing surface (277).

6. The intravascular blood pump according to any one of the preceding claims, wherein the impeller bearing (37) is a sliding bearing, wherein preferably the impeller bearing (37) is a blood purged sliding bearing.

7. The intravascular blood pump according to any one of the preceding claims, wherein a center of area of the primary blood flow inlet (211) is arranged in the separated secondary blood flow inlet (212).

8. The intravascular blood pump according to any one of the preceding claims, wherein one secondary blood flow inlet (212) of the at least one secondary blood flow passage (321) is arranged at a position of the axis of rotation (10).

9. The intravascular blood pump according to any one of the preceding claims, wherein a secondary channel intake (324) forming part of the at least one secondary blood flow passage (321) is arranged upstream of a primary channel intake (314) forming part of a primary impeller channel (311) in the primary blood flow passage (30).

10. The intravascular blood pump according to any one of the preceding claims, wherein the primary impeller (31) comprises at least one blade (313) which has a primary pitch at the upstream end of the primary impeller (31) and wherein the at least one secondary blood flow passage (321) has a secondary pitch, the secondary pitch being approximately or exactly the same as the primary pitch.

11. The intravascular blood pump according to any one of the preceding claims, wherein exactly one of the at least one secondary blood flow passage (321) has an upstream end arranged at a position of the axis of rotation (10).

12. The intravascular blood pump according to any one of the preceding claims, wherein at least two of the at least one secondary blood flow passage (321) are arranged asymmetrically in regard to the axis of rotation (10) and/or wherein two of the at least one secondary blood flow passage (321) are arranged opposite to each other in regard to the axis of rotation (10).

13. The intravascular blood pump according to any one of the preceding claims, comprising exactly two of the at least one secondary blood flow passage (321).

14. The intravascular blood pump according to any one of the preceding claims, wherein the primary impeller (31) comprises an inlay in which the at least one secondary blood flow passage (321) is formed.

15. The intravascular blood pump according to any one of the preceding claims, comprising a drive unit (4) configured to rotate the primary impeller (31) about an axis of rotation (10).
